# EUROPEAN PATENT APPLICATION

(11) **EP 4 834 326 A1**
(43) Date of publication of application: **04.11.2026**
(21) Application number: 24911449.7
(22) Date of filing: 27.12.2024
(51) Int. Cl.: A61K 9/08, A61K 31/554, A61K 47/00, A61K 47/10, A61K 47/18, A61K 47/36, A61P 25/18

(54) **SOLUTION-TYPE QUETIAPINE COMPOSITION FOR TRANSNASAL ADMINISTRATION AND USE THEREOF**

(30) Priority: 29.12.2023 CN 202311871438
(71) Applicant: Sichuan Purity Pharmaceutical Co., Ltd., Chengdu, Sichuan 610096 (CN)
(72) Inventor: HAN, Miao, Chengdu, Sichuan 610095 (CN); ZHANG, Wenlong, Chengdu, Sichuan 610095 (CN); WANG, Wan, Chengdu, Sichuan 610095 (CN); SHI, Xinyue, Chengdu, Sichuan 610095 (CN); ZHANG, Bin, Chengdu, Sichuan 610095 (CN); WANG, Zhenyu, Chengdu, Sichuan 610095 (CN)
(74) Representative: Kador & Partner Part mbB
(86) International application number: PCT/CN2024/143117
(87) International publication number: WO 2025/140529

(57) **Abstract**

The present disclosure pertains to the technical field of pharmaceutical preparations, and provides a novel solution-type quetiapine composition for intranasal administration. In the provided composition, quetiapine is completely dissolved therein in the form of a solution, and administered intranasally. Compared with the commercially available oral dosage forms, the provided solution-type quetiapine intranasal preparation has particularly useful pharmacokinetic and pharmacodynamic profiles. Compared with other disclosed forms such as intranasal nano-emulsion forms, the composition of the present disclosure still has surprising pharmacokinetic and pharmacodynamic profiles, exhibits promising clinical application prospects, and is expected to address unmet clinical needs. On this basis, the present disclosure further provides an improved high-saturation solubility technical solution and an improved technical solution of good chemical stability for the preparation of the solution-type quetiapine intranasal preparation.

## Description

### TECHNICAL FIELD

The present disclosure pertains to the technical field of pharmaceutical preparations, in particular to the field of quetiapine pharmaceutical preparations, and more specifically to a novel composition for intranasal administration and use thereof.

### BACKGROUD

Quetiapine is a new atypical drug for the treatment of mental diseases. The action mechanism by which quetiapine exerts its therapeutic effect may involve antagonism of central D2 and 5-HT2A receptors. Around 1997, a tablet form of quetiapine was first marketed by AstraZeneca in the United States. The currently approved indications of the marketed tablet include "schizophrenia, depressive episodes of bipolar disorder, manic or mixed episodes of bipolar I disorder", with strengths of 25 mg, 50 mg, 100 mg, 200 mg, 300 mg, and 400 mg. Around 2009, a sustained-release (SR) tablet form of quetiapine was launched in the United States. The approved indications of the SR tablet include "schizophrenia, depressive episodes of bipolar disorder, manic or mixed episodes of bipolar I disorder, and adjunctive treatment of major depressive disorder", with strengths of 50 mg, 150 mg, 200 mg, 300 mg and 400 mg.

The quetiapine tablet and sustained-release tablet on the market are both oral formulations, and thus suffer from common clinical drawbacks, such as poor compliance, slow onset of dissolution and absorption due to first-pass effects, and lower bioavailability. Particularly for the target patients with mental diseases, these drawbacks often become unacceptable in clinical application of the drug.

Therefore, there is still an unmet clinical need for improvement of the currently marketed dosage forms of tablet and sustained-release tablet. Despite such a clear clinical need and the fact that the oral quetiapine has a bioavailability of only about 10%, no improved preparation with promising prospects has reached the market for more than 20 years since quetiapine entity was launched, probably owing to its inherent pharmaceutical properties (e.g., poor solubility). Moreover, over the past 20 years, most of the published research on quetiapine in the industry has focused on its chemical synthesis, and the preparation methods of the tablet, sustained-release tablet, dispersible tablet and the like, whereas reports on substantial improvements to address the clinical drawbacks of oral formulations are rare. .

A Chinese Patent as exemplified by CN102552128B provides an improved injection form. By formulating quetiapine as an injection, first-pass effect can be bypassed, the dosage can be reduced, and the clinical safety risks of adverse drug reactions and high-dose medication can be mitigated. Nevertheless, considering that quetiapine is used for patients with mental diseases, which often accompanied by acute and manic characteristics, and the discomfort (such as pain) caused by injection at that time may aggravate patients' nervous feelings and trigger emergency responses. Besides, injection administration also requires higher medical skills, making it inconvenient for patients to self-administration and limiting its applicable scenarios.

An Indian patent application as exemplified by IN202211053155A provides a nano-emulsion in which quetiapine is dissolved in an oil phase and then formulated into an oil-in-water nanoemulsion. The nanoemulsion is designed for targeted brain delivery via the olfactory pathway in the nasal cavity, thereby bypassing metabolism caused by the hepatic first-pass effect and improving bioavailability. The "Bioavailability and biodistribution studies" section of this patent shows that compared to oral formulations, the Cₘₐₓ and the AUC₀₋₄₈₀ after intranasal administration of the provided nanoemulsion are both significantly increased (Table. 11).

However, even with this nanoemulsion design, quetiapine dissolved in the oil phase and must pass through the oil phase to reach the aqueous phase during absorption after administration. Furthermore, since the saturation solubility of quetiapine in the aqueous phase is low, the time of the nanoemulsion to peak concentration and onset of action are prolonged. For the same reason as with oral formulations, this prolongation in time to peak concentration and onset of action is unfavorable for the rapid relief of some acute symptoms of mental diseases. The characteristics of this nanoemulsion is also documented in the "In-Vitro Drug Release Studies" section of the Indian patent, as shown in Table 10, FIG. 6, and its analysis conclusions, "diffusion from oily core and across interface is hindered by the aqueous medium, and the quetiapine, due to its low saturation solubility in water, poses a barrier to drug transport". Similarly, as shown in Table 11 of the "Bioavailability and biodistribution studies" section of the Indian patent, the intranasal nano-emulsion dosage form exhibits the same time to peak plasma concentration Tₘₐₓ = 2 hours as the oral preparation (both being much longer than 0.25 hour for injection), which is also consistent with the data and conclusions in the aforementioned in vitro studies.

From the above, it can be seen that in the field of quetiapine research, first, there are few studies on substantial improvements aimed to address the clinical drawbacks of the marketed oral formulations. Second, the improved dosage forms provided in these rarely disclosed literatures still fail to overcome the clinical drawbacks similar to those of oral formulations. Against this backdrop, there remains an urgent need for an improved form of quetiapine that addresses the drawbacks of the marketed oral dosage forms and meets the unmet critical clinical need.

### SUMMARY

To solve the above issues existing in the prior art, the primary object of the present disclosure is to provide an improved form of quetiapine, which, compared with the commercially available oral dosage forms, should have a rapid time to peak concentration or onset of action, ease of administration and high patient compliance. In addition, such a novel improved form also should have a higher bioavailability so as to avoid the clinical safety risks associated with the high dosages required by the oral formulations due to its low bioavailability.

For the primary object, the present disclosure provides the following technical solution based on extensive attempts.

A quetiapine composition for intranasal administration, comprising quetiapine or a pharmaceutically acceptable salt thereof, wherein the composition is a solution. That is, the improved form provided in the present disclosure is a "solution-type intranasal administration composition". After extensive research and exploration, it is surprisingly found that preparing quetiapine into a solution and administering it intranasally can result in a significantly shortened time to peak concentration and onset of action, for example, a remarkable reduction in Tₘₐₓ. Furthermore, preparing quetiapine into a solution and administering it intranasally can also achieve a significantly enhanced bioavailability, which is not attainable with conventional improvements.

For the solution-type quetiapine composition for intranasal administration provided in the present disclosure:
In some embodiments, the mass percentage of quetiapine in the composition, calculated as free base, is from 0.22% to 11.7% or from 4.3% to 11.7%. Alternatively, preferably, quetiapine is dissolved in the composition in the form of fumarate, and the mass percentage of quetiapine fumarate in the composition is from 0.25% to 13.4%, from 0.25% to 5%, from 0.25% to 1.7%, from 5% to 13.4%, from 5% to 9.7%, from 5% to 7%, 0.25%, 1.7%, 5%, 5.2%, 6%, 6.5%, 6.6%, 6.7%, 7%, 8.2%, 9.5%, 9.7%, 13%, 13.3% or 13.4%.

In other embodiments, the solution-type quetiapine composition for intranasal administration further comprises one or a combination of two or more selected from a solubilizer, a cosolvent, a viscosity enhancer, a stabilizer, a preservative, a pH regulator and a pH buffer. The solubilizer optionally comprises one or a combination of two or more selected from benzyl alcohol, DMA, cyclodextrin and a surfactant, wherein the cyclodextrin optionally comprises one or a combination of two or more selected from β-cyclodextrin, hydroxypropyl-β-cyclodextrin and sulfobutyl-β-cyclodextrin, and wherein the surfactant optionally comprise one or a combination of two or more selected from Tween, Span, Carbomer and polyethylene glycol-15 hydroxystearate. The cosolvent optionally comprises saturated alcohol, and the saturated alcohol optionally comprises one or a combination of two or more selected from propylene glycol, ethanol and glycerol. The viscosity enhancer optionally comprises one or a combination of two or more selected from polyethylene glycol, pectin, cellulose, polyolefin and polyacrylic acid. The stabilizer optionally comprises edetate, and the edetate optionally comprises EDTA-2Na and/or EDTA-2K. The preservative optionally includes benzalkonium chloride, benzalkonium bromide, benzethonium chloride, potassium sorbate, methyl paraben, ethyl paraben, propyl paraben, butyl paraben, isopropyl paraben, isobutyl paraben, sodium propyl paraben and/or sodium methyl paraben. The pH regulator optionally comprises one or a combination of two or more selected from citric acid, hydrochloric acid, sodium hydroxide, potassium hydroxide and meglumine. The pH buffer optionally comprises one or a combination of two or more selected from citrate buffer, phosphate buffer and acetate buffer.

In other embodiments, the solution-type quetiapine composition for intranasal administration further comprises a solubilizer, a cosolvent, a viscosity enhancer and water. The solubilizer is benzyl alcohol, and the mass percentage of benzyl alcohol in the composition is selected from 1% to 10%, from 1% to 9.3%, from 5% to 9.3%, 1%, 3.2%, 5%, 7.5% or 9.3%. The cosolvent is one or a combination of two or more selected from propylene glycol, ethanol and glycerol, preferably propylene glycol, wherein the mass percentage of propylene glycol in the composition is selected from 0% to 12%, from 4.6% to 12%, from 6% to 12%, 4.6%, 4.7%, 5%, 6%, 10%, 11% or 12%, the mass percentage of ethanol in the composition is selected from 0% to 20%, from 6% to 20%, from 0% to 19.8%, from 18.3% to 19.8%, 6%, 10%, 18.3%, 18.5% or 19.8%, and the mass percentage of glycerol in the composition is selected from 0% to 5%, from 0% to 3%, from 0% to 2.5%, from 0% to 2.2% or 2.2%. The viscosity enhancer is polyethylene glycol, preferably PEG 3350 or PEG 400, the mass percentage of PEG 3350 in the composition is selected from 0% to 20%, from 2.5% to 15%, from 5% to 15%, 2.5%, 4.6%, 4.7%, 5%, 15% or 20%, and the mass percentage of PEG 400 in the composition is selected from 0% to 15%, 10% or 15%.

In other embodiments, the solution-type quetiapine composition for intranasal administration further comprises one or a combination of two or more selected from a stabilizer, a preservative, a pH regulator and a pH buffer. The stabilizer is EDTA-2Na, and the mass percentage of EDTA-2Na in the composition is selected from 0% to 0.1% or 0.05%. The preservative is benzalkonium chloride, and the mass percentage of benzalkonium chloride in the composition is selected from 0% to 0.02% or 0.02%. The pH regulator is one or a combination of two or more selected from citric acid, hydrochloric acid, sodium hydroxide and meglumine, and the pH regulator adjusts the pH value of the composition to 4.1-6.0, 4.5-6.0, 5.0-6.0, or to 4.1, 4.5, 4.9, 5.0, 5.1, 5.3 or 6.0. The pH buffer is a citrate buffer, a phosphate buffer or an acetate buffer.

The second object of the present disclosure is, based on the solution-type intranasal administration composition provided in the above primary object, to increase the saturation solubility of quetiapine in the solution-type composition.

Limited by the area of nasal mucosa, the volume per dose in intranasal administration cannot be too high. A dosing volume that is too high would result in surplus liquid that cannot be absorbed by the nasal mucosa, and the surplus liquid will flow out of the nasal cavity or even enter the throat via nasopharynx. This results not only in a failure of absorption and therapeutic effect but also leads to an unpleasant administration experience and unnecessary side effects. A single-dose volume for intranasal spray administration is typically not more than 200 µL, and preferably maintained at no more than 100 µL. However, when delivering a drug in a "solution-type" form with such a small volume, it is necessary for the active ingredients to have a high dissolved concentration so as to ensure the administration dosage. Meanwhile, a high drug concentration will avoid multiple administrations, and thus reduce irritation to the nasal cavity. Testing has shown that quetiapine is a drug with low saturation solubility, and quetiapine fumarate is present in water at a saturation solubility of only 4.0 mg/mL. Therefore, in order to ensure the dosage of administration and alleviate nasal irritation by reducing number of sprays, it is highly necessary to increase the saturation solubility of quetiapine by variable means.

The second object of the present disclosure is achieved by the following technical solution:
A intranasal administration composition with improved dissolved concentration of quetiapine comprises quetiapine or a pharmaceutically acceptable salt thereof. The composition is a solution and further comprises a solubilizer. The solubilizer optionally comprises one or a combination of two or more selected from benzyl alcohol, DMA, cyclodextrin and surfactant, preferably benzyl alcohol or DMA.

The present disclosure has found in further research and exploration that for quetiapine with poor solubility, it is relatively easy to increase the saturation solubility to about 20 mg/mL by using a common solubilization method. However, further increasing the saturation solubility of quetiapine beyond this range becomes more difficult, particularly increasing the solubility to 50 mg/mL or higher is significantly challenging. Nevertheless, in order to deliver a therapeutic dose of the drug at a small dosing volume, it is beneficial or even necessary to increase the drug concentration to more than 50 mg/mL. During the extensive testing for the present disclosure, it was surprisingly found that the use of solubilizer benzyl alcohol or DMA is highly beneficial to increase the saturation solubility of quetiapine to a higher concentration that is 50 mg/mL or more.

In particular, the application of benzyl alcohol as a solubilizer yields an especially unexpected beneficial effect, which not only significantly improves the saturation solubility of quetiapine, but also offers lower nasal mucosa irritation than other solubilizers such as DMA (benzyl alcohol is also an inactive ingredient excipient included in the "FDA Drug Inactive Ingredient Database" and is allowed to the intranasal spray administration route, and its irritation and safety of nasal route have been recognized by regulatory verification). These make it highly suitable as a solubilizer for the solution-type quetiapine composition for intranasal administration provided in the present disclosure. In addition, it is surprising that benzyl alcohol is widely used as a preservative and disinfectant in the industry, whereas in the solution-type quetiapine composition for intranasal administration according to the present disclosure, the addition of benzyl alcohol unexpectedly provides significant beneficial assistance in increasing saturation solubility of quetiapine. Therefore, benzyl alcohol is used as the most preferred solubilizer of the solution-type quetiapine composition for intranasal administration provided in the present disclosure. On this basis, in other embodiments, the solubilizer in the solution-type intranasal administration composition with improved dissolved concentration of quetiapine does not contain other types of solubilizers which are more irritating to the nasal cavity than benzyl alcohol. As several specific options, the solubilizer in the composition may only comprise benzyl alcohol, or the solubilizer in the composition may only comprise benzyl alcohol and cyclodextrin, or the solubilizer in the composition may only comprise benzyl alcohol and a surfactant, or the solubilizer in the composition may only comprise benzyl alcohol, cyclodextrin and a surfactant.

In other embodiments, as illustrated by the specific examples provided herein, the mass percentage of benzyl alcohol in the composition according to the second object may be selected from 1% to 10%, from 3.2% to 10%, from 5% to 10%, from 5% to 9.3%, from 5% to 7.5%, 1%, 3.2%, 5%, 7.5%, 9.3% or 10%. Depending on the amount of quetiapine added to the composition, the amount of benzyl alcohol added may fluctuate within ±20% of the amount of quetiapine, that is, preferably, the mass ratio of benzyl alcohol to quetiapine in the composition is 1: 0.8 to 1: 1.2.

In other embodiments, the solution-type intranasal administration composition with improved dissolved concentration for quetiapine according to the present disclosure further comprises a cosolvent, and the cosolvent optionally comprises saturated alcohol. The saturated alcohol optionally comprises one or a combination of two or more selected from propylene glycol, ethanol, and glycerol, preferably propylene glycol or a combination of propylene glycol and ethanol. An addition of an additional cosolvent, such as propylene glycol, can promote the dissolution of poorly soluble drugs, and thus facilitate a further increase in the saturation solubility of quetiapine in the provided composition. Among them, as illustrated by the specific examples provided herein, the mass percentage of propylene glycol in the composition may be selected from 0% to 12%, from 4.6% to 12%, from 6% to 12%, 4.6%, 4.7%, 5%, 6%, 10%, 11% or 12%. The mass percentage of ethanol in the composition may be selected from 0% to 20%, from 6% to 20%, from 0% to 19.8%, from 18.3% to 19.8%, 6%, 10%, 18.3%, 18.5% or 19.8%. The mass percentage of glycerol in the composition may be selected from 0% to 5%, from 0% to 3%, from 0% to 2.5%, from 0% to 2.2% or 2.2%.

In other embodiments, the solution-type intranasal administration composition with improved dissolved concentration of quetiapine further comprises a surfactant and/or polyethylene glycol, and preferably further comprises one or a combination of two or more selected from Tween, PEG 3350 and PEG 400, wherein the Tween comprises Tween 80 or Tween 20. The addition of these additional substances can further stabilize the solution system, particularly inhibiting the unexpected precipitation of quetiapine under high- or low-temperature conditions. Among them, as illustrated by the specific examples provided herein, the mass percentage of PEG 3350 in the composition may be selected from 0% to 20%, from 2.5% to 15%, from 5% to 15%, 2.5%, 4.6%, 4.7%, 5%, 15% or 20%. The mass percentage of PEG 400 in the composition may be selected from 0% to 15%, 10% or 15%. The mass percentage of Tween in the composition may be selected from 0% to 1.5%, 0.5%, 1%, 1.4% or 1.5%.

In other embodiments, the solution-type intranasal administration composition with improved dissolved concentration of quetiapine according to the present disclosure further comprises cyclodextrin, the cyclodextrin comprises one or a combination of two or more selected from β-cyclodextrin, hydroxypropyl-β-cyclodextrin and sulfobutyl-β-cyclodextrin, and the mass percentage of the cyclodextrin in the composition may be selected from 0% to 10%, from 1% to 5%, or 5%. Cyclodextrin provides a certain improvement in the saturation solubility of the solution-type quetiapine composition, but its single use is not as effective as benzyl alcohol or DMA.

In other embodiments, the dissolved concentration of quetiapine in the solution-type intranasal administration composition with improved dissolved concentration of quetiapine according to the present disclosure, calculated as free base, is from 4.3% to 11.7%. In particular, the dissolved concentration of quetiapine free base is from 5.6% to 11.7% by mass. As illustrated by the specific examples provided herein, quetiapine is preferably dissolved in the composition in the form of fumarate, and the mass percentage of quetiapine fumarate in the composition is from 5% to 13.4%, 5% to 9.7%, 5% to 7%, 5%, 5.2%, 6%, 6.5%, 6.6%, 6.7%, 7%, 8.2%, 9.5%, 9.7%, 13%, 13.3% or 13.4%. A higher concentration of the active ingredient is particularly suitable for intranasal spray administration, which can significantly reduce the number of intranasal sprays in a single treatment, thereby reducing frequent irritation to the nasal cavity and alleviating clinical medication discomfort and side effects such as sneezing and tingling.

In other embodiments, the solution-type intranasal administration composition with improved dissolved concentration of quetiapine according to the present disclosure is an aqueous solution, which further comprises water and one or a combination of two or more selected from a viscosity enhancer, a stabilizer, a preservative, a pH regulator and a pH buffer. It should be noted that these optional excipients of "a viscosity enhancer, a stabilizer, a preservative, a pH regulator and a pH buffer" mentioned in the second object of the present disclosure may be selected in any combination with reference to the types and dosage of corresponding excipients mentioned anywhere in the aforementioned primary object of the present disclosure.

The third object of the present disclosure is to further provide a composition with good chemical stability based on the solution-type quetiapine composition for intranasal administration provided in the first and second objects.

The third object of the present disclosure is achieved by the following technical solution:
A stable intranasal administration composition, wherein the composition is a solution including water and the following components:

| **Component** | **Mass Percentage** |
|---|---|
| Quetiapine or a pharmaceutically acceptable salt thereof (calculated as free base) | 0.22%-11.7% |
| Solubilizer | 1%-10% |
| Cosolvent | 0%-20% |
| Viscosity enhancer | 0%-15% |

.

In some embodiments, the mass percentage of quetiapine in the stable solution-type quetiapine composition for intranasal administration, calculated as free base, is from 4.3% to 11.7%. As illustrated by the specific examples provided herein, the quetiapine is preferably dissolved in the composition in the form of fumarate, and the mass percentage of quetiapine fumarate in the composition is from 0.25% to 13.4%, from 0.25% to 5%, from 0.25% to 1.7%, from 5% to 13.4%, from 5% to 9.7%, from 5% to 7%, 0.25%, 1.7%, 5%, 5.2%, 6%, 6.5%, 6.6%, 6.7%, 7%, 8.2%, 9.5%, 9.7%, 13%, 13.3% or 13.4%.

In other embodiments, the solubilizer in the stable solution-type quetiapine composition for intranasal administration comprises one or a combination of two or more selected from benzyl alcohol, cyclodextrin, Tween and polyethylene glycol-15 hydroxystearate, the cyclodextrin optionally comprises hydroxypropyl-β-cyclodextrin, and the Tween optionally comprises Tween 80 and/or Tween 20. As illustrated by the specific examples provided herein, the mass percentage of benzyl alcohol in the composition may be selected from 1% to 10%, from 1% to 9.3%, from 5% to 9.3%, 1%, 3.2%, 5%, 7.5% or 9.3%, the mass percentage of cyclodextrin in the composition may be selected from 0% to 5% or 5%, the mass percentage of Tween in the composition may be selected from 0% to 1.4%, 1% or 1.4%, and the mass percentage of polyethylene glycol-15 hydroxystearate in the composition may be selected from 0% to 3%, 1.5% or 3%.

In other embodiments, the cosolvent in the stable solution-type quetiapine composition for intranasal administration comprises one or a combination of two or more selected from propylene glycol, ethanol and glycerol. As illustrated by the specific examples provided herein, wherein the mass percentage of propylene glycol in the composition may be selected from 0% to 12%, from 4.6% to 12%, from 6% to 12%, 4.6%, 4.7%, 6%, 11% or 12%, the mass percentage of ethanol in the composition may be selected from 0% to 20%, from 0% to 19.8%, from 18.3% to 19.8%, 18.3%, 18.5% or 19.8%, and the mass percentage of glycerol in the composition may be selected from 0% to 5%, from 0% to 3%, from 0% to 2.5%, from 0% to 2.2% or 2.2%.

In other embodiments, the viscosity enhancer in the stable solution-type quetiapine composition for intranasal administration comprises polyethylene glycol and/or pectin, and the polyethylene glycol optionally comprises PEG 3350 and/or PEG 400. As illustrated by the specific examples provided herein, the mass percentage of PEG 3350 in the composition may be selected from 0% to 5%, 2.5% to 5%, 2.5%, 4.6%, 4.7% or 5%, the mass percentage of PEG 400 in the composition may be selected from 0% to 15% or 15%, and the mass percentage of the pectin in the composition may be selected from 0% to 1% or 0.7%.

In other embodiments, the stable solution-type quetiapine composition for intranasal administration further comprises any one or a combination of two or more selected from the following components:

| **Component** | **Mass Percentage** |
|---|---|
| Stabilizer | 0%-0.1% |
| Preservative | 0%-0.2% |
| pH regulator | An amount effective to adjust the pH of the composition to 4.1-6.9 |

In other embodiments, the stabilizer in the stable solution-type quetiapine composition for intranasal administration comprises edetate, the edetate comprises disodium edetate and/or dipotassium edetate, and the mass percentage of the disodium edetate in the composition may be selected from 0% to 0.1% or 0.05%. The preservative comprises benzalkonium chloride, and the mass percentage of benzalkonium chloride in the composition may be selected from 0% to 0.02% or 0.02%.

In other embodiments, the pH regulator in the stable solution-type quetiapine composition for intranasal administration comprises one or a combination of two or more selected from sodium hydroxide, potassium hydroxide, meglumine, citric acid and hydrochloric acid, and the pH regulator may be added in an amount effective to adjust the pH of the composition to 4.1-6.0, 4.5-6.0, 5.0-6.0, 4.1, 4.5, 4.9, 5.0, 5.1, 5.3 or 6.0.

In other embodiments, the mass percentage of meglumine in the stable solution-type quetiapine composition for intranasal administration may be from 0% to 0.5%, 0.02%, 0.25% or 0.5%, and the citric acid is optionally a 1 M citric acid solution.

In other embodiments, optionally, the stable solution-type quetiapine composition for intranasal administration is further added with or without a pH buffer, and the pH buffer optionally includes one or a combination of two or more selected from citrate buffer, phosphate buffer and acetate buffer.

In other embodiments, the stable solution-type quetiapine composition for intranasal administration is free of sodium sulfite and sodium bisulfite. As illustrated by the specific examples provided herein, the introduction of sodium sulfite and sodium bisulfite unexpectedly and significantly destroys the chemical stability of the formulation.

In other embodiments, the stable solution-type quetiapine composition for intranasal administration consists only of quetiapine fumarate, a solubilizer, a cosolvent, a viscosity enhancer and water, and does not contain other unspecified components except for pharmaceutically acceptable levels of impurities. The solubilizer is benzyl alcohol, the cosolvent is one or a combination of two or more selected from propylene glycol, ethanol and glycerol, particularly propylene glycol, and the viscosity enhancer is PEG 3350 and/or PEG 400. Optionally, a combination of two or more selected from benzalkonium chloride, a pH regulator and a pH buffer is optionally added or not added to the composition.

Based on the stable solution-type quetiapine composition for intranasal administration provided for the above objects, the present disclosure further provides a quetiapine nasal spray, including the solution-type quetiapine composition for intranasal administration according to any one of the foregoing aspects of the present disclosure and a quantitative nasal spray device for applying and delivering the composition into nasal cavity, wherein a volume of the composition delivered per spray by the quantitative nasal spray device may be selected from 25 µL to 200 µL, from 50 µL to 150 µL, 25 µL, 50 µL, 75 µL, 100 µL, 125 µL, 150 µL, 175 µL or 200 µL.

Accordingly, based on the clinical value of quetiapine that has been discovered and verified by the animal experiments provided herein, the present disclosure further provides use of the solution-type quetiapine composition for intranasal administration according to any one of the foregoing aspects of the present disclosure or the quetiapine nasal spray of the present disclosure described above in the manufacture of a medicament for treating mental diseases.

### DESCRIPTION OF THE DRAWINGS

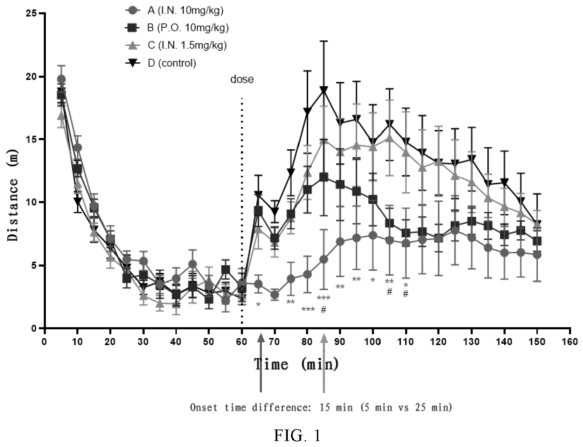

FIG. 1 shows the movement distance of experimental rats every 5 minutes from 0 to 150 minutes after the start of the test in Example 2 of Example A. Data are expressed as Mean ± SEM. Two-way analysis of variance was adopted (for the comparison of test substance A and test substance D, * indicates p < 0.05, ** indicates p < 0.01, *** indicates P < 0.001; and for the comparison of test substance B and test substance D, # indicates p < 0.05).

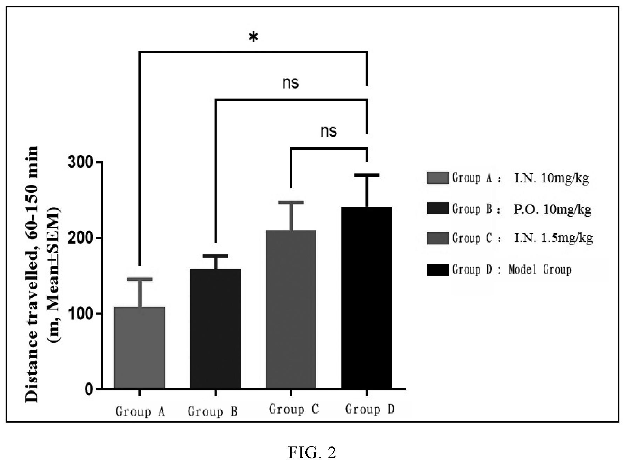

FIG. 2 shows the total movement distance of experimental rats from 60 to 150 minutes after the start of the test in Example 2 of Example A. Data are expressed as Mean ± SEM. One-way analysis of variance was adopted (for the comparison of test substance A and test substance D, * indicates p < 0.05).

### DESCRIPTION OF EXEMPLARY EMBODIMENTS

Unless otherwise specified, all percentage "%" appearing anywhere in the present disclosure refers to the percentage by mass.

In the present disclosure, to more conveniently estimate the saturation solubility, the density of the solution composition is approximated as 1 g/mL.

In the present disclosure, the conversion factor between quetiapine fumarate and quetiapine free base is 1.15, that is, "the amount of quetiapine free base = the amount of quetiapine fumarate / 1.15".

In the present disclosure, the balance means adding corresponding components to 100%.

The meanings of the abbreviations in the present disclosure are in accordance with the common understanding of the industry, and some terms are annotated as follows:
I.N.: intranasal administration;
P.O.: oral administration;
I.V.: intravenous injection;
I.P.: intraperitoneal injection;
HS-15: polyethylene glycol-15 hydroxystearate;
HP-β-CD: hydroxypropyl-β-cyclodextrin;
PEG 3350: polyethylene glycol 3350;
PEG 400: polyethylene glycol 400;
EDTA: edetate;
EDTA-2Na: disodium edetate, also known as disodium ethylenediaminetetraacetate;
EDTA-2K: dipotassium edetate, also known as dipotassium ethylenediaminetetraacetate;
DMA: N,N-dimethylacetamide.

The present disclosure will be further explained below in conjunction with the accompanying drawings and specific examples.

### Example A: Animal pharmacokinetic study and pharmacodynamic study on the solution-type intranasal administration composition of the present disclosure

### Example 1 Pharmacokinetic study in beagle dogs

### 1. Purpose of the experiment

The purpose of this experiment is to evaluate and compare the pharmacokinetic profiles of the solution-type intranasal administration composition of the present disclosure with those of oral tablets and injections after administration to beagle dogs.

### 2. Test substances

The codes for the types of test substances are as follows:

| **Code** | **Group** | **Drug Concentration (calculated as free base)** | **Administration Mode** | **Administration Volume** | **Administration Dose** |
|---|---|---|---|---|---|
| A | Nasal spray 1 | 92.5 mg/mL | Intranasal spray | 100 µL/spray, 3 sprays in total | 27.75 mg/dog |
| B | Nasal spray 2 | 92.3 mg/mL | Intranasal spray | 100 µL/spray, 3 sprays in total | 27.75 mg/dog |
| C | Quetiapine fumarate tablet | 25 mg/tablet | Oral | 1 tablet/dog | 25 mg/dog |
| D | Injection | 2.5 mg/mL | Intravenous injection | 1 mL/kg | 25 mg/dog |

The formulation compositions of test substances A and B are as follows:

| | **Quetiapine fumarate** | **Benzyl alcohol** | **Propylene glycol** | **PEG 3350** | **Tween 20** | **Pectin** | **Anhydrous ethanol** | **0.1 M citric acid solution** |
|---|---|---|---|---|---|---|---|---|
| A | 10.6% | 9.3% | 4.7% | 4.7% | 1.4% | / | 18.5% | Balance |
| B | 10.6% | 9.3% | 4.6% | 4.6% | 1.4% | 0.7% | 18.3% | Balance |
| "/" indicates no addition of the corresponding component in the preparation. | | | | | | | | |

Preparation method for the test substances A and B: the individual components were weighed, mixed uniformly, dissolved to clarify, then filled into a medium borosilicate glass vial, and assembled with a quantitative nasal spray pump to obtain the final product.

The quetiapine fumarate tablet in Group C was purchased from Hunan Dongting Pharmaceutical Co., Ltd. through a regular commercial channel.

The injection in Group D was prepared by dissolving quetiapine fumarate in normal saline.

### 3. Experimental method

### 3.1 Experimental animals

Eight healthy adult Beagle dogs, male, weighing between 6.5 kg and 9.5 kg.

### 3.2 Experimental administration

The animals were randomly divided into 4 groups with 2 dogs per group. Except for one random group which was administered the injection of test substance D via intravenous injection, the other three groups were administered test substances A, B and C in a crossover manner over a total of three periods. A 7-day washout was implemented prior to switching the test substance in each cycle.

Taking into account the tablet strength and the saturation solubility of the nasal spray, the dosage in this test was set at 25 mg per dog for the oral group and the intravenous injection group, and 27.75 mg per dog for the nasal spray group. When calculating pharmacokinetic parameters, the doses were normalized to an equivalent dose for comparison.

### 3.3 Blood sample collection and processing

1 mL of blood was collected from the forelimb vein and anticoagulated with EDTA-K2 (the blood sample was placed in an ice bath after collection), then centrifuged at 3200 g for 10 minutes at 4°C within 2 hours to separate the plasma, and the plasma was stored at -80°C until testing.

### Sampling time point:

Oral group C: before administration (0 hours), and 25 minutes, 45 minutes, 1 hour, 1.5 hours, 2 hours, 4 hours, 6 hours, 8 hours and 12 hours after administration.

Nasal spray groups A and B, and intravenous injection group D: before administration (0 hours), and 5 minutes, 10 minutes, 15 minutes, 30 minutes, 45 minutes, 1 hour, 4 hours, 8 hours and 12 hours after administration.

### 3.4 Analysis method and results

The drug concentration in the plasma of Beagle dogs at different time points after administration was measured, and the plasma concentration-time curves were plotted. The main pharmacokinetic parameters were calculated using WinNonlin 8.3 software as follows:

| | **Cₘₐₓ** | **Tₘₐₓ** | **F** |
|---|---|---|---|
| **A Nasal spray 1** | 1343 ± 492 ng/mL | 0.0973 ± 0.0342 h | 104 ± 28% |
| **B Nasal spray 2** | 1374 ± 768 ng/mL | 0.125 ± 0.070 h | 99.5 ± 32.2% |
| **C Quetiapine fumarate tablet** | 70.7 ± 43.8 ng/mL | 0.889 ± 0.414 h | 16.3 ± 7.5% |

The results demonstrated that the solution-type nasal spray preparation of the present disclosure exhibited a significantly shortened time to peak Tₘₐₓ compared to the marketed tablet, and the maximum plasma concentration Cₘₐₓ and bioavailability F were significantly increased by approximately 20-fold and 6-fold, respectively.

### Example 2 Pharmacodynamic study in a rat model of hyperlocomotion induced by methamphetamine

### 1. Purpose of the experiment

The purpose of this experiment is to evaluate and compare the pharmacodynamic effects of the solution-type intranasal administration composition of the present disclosure with those of oral quetiapine preparation in the rat model of hyperlocomotion induced by methamphetamine.

### 2. Test substances

The codes for the types of test substances are as follows:

| **Code** | **Group** | **Drug Concentration** | **Administration Mode** | **Administration Volume** | **Administration Dose** |
|---|---|---|---|---|---|
| A | High-dose intranasal group | 60 mg/mL | Intranasal | 0.167 mL/kg | 10 mg/kg |
| B | Oral group | 3 mg/mL | Intragastric | 3.333 mL/kg | 10 mg/kg |
| C | Low-dose intranasal group | 60 mg/mL | Intranasal | 0.025 mL/kg | 1.5 mg/kg |
| D | Vehicle control group | 0 mg/mL | Intranasal | 0.167 mL/kg | 0 mg/kg |

The formulation compositions of the test substances are as follows:

| **Code** | **Quetiapine fumarate** | **Benzyl alcohol** | **Propylene glycol** | **PEG 3350** | **Purified water** |
|---|---|---|---|---|---|
| A | 7% | 7.5% | 12% | 2.5% | Balance |
| B | 0.35% | 0.5% | 12% | 2.5% | Balance |
| C | 7% | 7.5% | 12% | 2.5% | Balance |
| D | 0% | 7.5% | 12% | 2.5% | Balance |

Preparation method for each test substance: the individual components were weighed, mixed uniformly and dissolved until clear to obtain the final product.

### 3. Experimental method

### 3.1 Experimental animals

Forty 7-week-old SD rats, with 10 rats in each group, were randomly divided into 4 groups, corresponding to each test substance respectively. The rats were acclimated to the experimental environment for one day prior to the test, and brought into the laboratory one hour in advance on the test day for further acclimatization.

### 3.2 Experimental administration

On the test day, animals in different group were administered test substances A, B, C or D in conscious state via corresponding modes of administration. Immediately after administration, methamphetamine modeling was performed (via intraperitoneal injection). The methamphetamine was administered at a dose of 3 mg/kg to induce hyperactivity in the animals.

### 3.3 Phenomena, locomotor activity testing and recording

Any-maze software recorded the distance the animals moved every five minutes to assess the levels of locomotor activity.

Before administration, the animals were placed in an observation box to detect their locomotor activity for 60 minutes.

After administration according to their groups, the animals were returned to the observation box and continuously observed and recorded for 90 minutes. The distance (in meters) the animals moved in the observation box was counted every 5 minutes, and abnormal phenomena such as toxic reactions or deaths of the animals during the experiment were promptly recorded.

On the day following the experiment, a cage-side observation was conducted to record the status of the experimental animals in each group, and to determine whether the activity level returned to normal, whether there were animals suffering from hyperactivity or hypoactivity, and whether there were the phenomena such as toxic reactions.

### 3.4 Data collection

Statistical analysis was performed using Prism software. Two-way ANOVA followed by Bonferroni's multiple comparisons test was used to compare the locomotor distance of animals in each group at different time points, while one-way ANOVA was applied to compare the total locomotor distance. A p-value < 0.05 was considered statistically significant.

### 4. Experimental results

The data are as illustrated in FIG. 1.

From 0 to 60 minutes after the start of the test, there was no statistical difference in the total distance moved among the four groups of animals.

After 60 minutes of acclimation, drug administration was started.

From 65 to 110 minutes after the start of the test, the high-dose intranasal group of test substance A significantly reduced the distance the animals moved every 5 minutes compared with the control group of test substance D.

At 85 minutes and from 105 to 110 minutes after the start of the test, the distance moved by the animals in every 5 minutes was significantly reduced in the oral group of test substance C compared with the control group of test substance D.

The data are as illustrated in FIG. 2.

From 60 to 150 minutes after the start of the test, the total distance moved by the animals in the high-dose intranasal group of test substance A was significantly reduced compared with the control group of test substance D. However, there was no significant difference in the total distance moved by the animals in the oral group of test substance C compared with the blank group of test substance D.

Cage-side observations during the experiment and on the day following the experiment showed no decrease in body weight, no adverse mental status, and no abnormal activity in the animals.

The results demonstrated that the high-dose intranasal group significantly inhibited the methamphetamine-induced hyperlocomotor activity in rats, including the distance moved per 5 minutes and the total distance moved, and provided a significant advantage in rapid onset of action, and prolonged, stable inhibition. The oral group was able to inhibit hyperlocomotor activity only at certain time points, with a slower onset, weaker degree of inhibition, and shorter duration. Moreover, there was no significant difference in total movement distance from the control group. Furthermore, the intranasal group also exhibited favorable safety profiles.

### Example B: Study on the solubilization of quetiapine in the solution-type intranasal administration composition of the present disclosure

### 1. Determination of saturation solubility

The present disclosure first tested the saturation solubility of quetiapine fumarate in common solvents, as follows:

| **Solvent** | **Saturation solubility (mg/mL)** |
|---|---|
| Water | 4.0 |
| Anhydrous ethanol | 4.6 |
| Propylene glycol | 8.8 |
| PEG 400 | 4.9 |
| Phosphate buffer, pH 4.5 | 5.7 |
| Acetate buffer, pH 4.5 | 6.7 |
| Citrate buffer, pH 4.5 | 5.7 |
| Citrate buffer, pH 5.5 | 5.0 |
| Citrate buffer, pH 6.5 | 7.4 |

The above tests demonstrated that the saturation solubility of quetiapine fumarate in water was very low (4.0 mg/mL), and the saturation solubility in other common solvents such as ethanol was also low.

The saturation solubility was higher at a moderately reduced pH, and the optimal pH for the solution-type preparation ranged from 4.5 to 6.5. However, even if the pH value was controlled at a low level, the saturation solubility of quetiapine fumarate was still at a low level, which could not meet the requirements of the solution-type preparation for intranasal delivery.

### 2. Saturation solubility with the addition of ethanol, propylene glycol, PEG 400, Tween 20 and Span 20

The present disclosure further tested the following formulation solvents, with the saturation solubility of quetiapine fumarate as follows:

| **Solvent** | **Saturation solubility (mg/mL)** |
|---|---|
| 19.8 wt% ethanol | 11.7 |
| 12 wt% propylene glycol | 5.2 |
| 20 wt% PEG 400 | 7.6 |
| 2.5 wt% Tween 20 | 4.4 |
| 22.9 wt% Span 20 | 7.5 |

The experiment with the above formulation solvents demonstrated that although the addition of ethanol, propylene glycol, PEG 400, Tween 20, and Span 20 to the aqueous solution had a certain improvement on the saturation solubility of quetiapine fumarate, the improvement was limited and still failed to meet the requirements for the solution-type preparation for intranasal delivery.

### 3. Basic formulation design for solubilization and buffer screening

| **Preparation** | **B1 (acetate buffer pair)** | **B2 (phosphate buffer pair)** | **B3 (citrate buffer pair)** |
|---|---|---|---|
| Quetiapine fumarate | 5.2% | 5.2% | 5.2% |
| mPEG 350 | 20% | 20% | 20% |
| PEG 400 | 15% | 15% | 15% |
| Propylene glycol | 6% | 6% | 6% |
| Ethanol | 6% | 6% | 6% |
| Buffer, pH 4.5 | Balance | | |
| **Saturation solubility (mg/mL)** | **16.96** | **19.96** | **20.83** |
| Preparation method: the individual components were weighed according to the formulation table, mixed, and dissolved sufficiently but were not completely dissolved. After standing, the supernatant was separated and collected, and precipitation occurred approximately 3 hours later. After standing for 4 days, the saturation solubility of quetiapine in the supernatant was analyzed and determined (calculated as free base). | | | |

The experimental process and results demonstrated that the above preparations had a certain improvement on the saturation solubility of quetiapine fumarate, but the improvement was limited and still failed to meet the requirements for the solution-type preparation for intranasal delivery. It also demonstrated that different types of buffer salts had no obvious effect on saturation solubility, but exerted a certain influence on dissolution rate, with citric acid buffer pair being relatively superior.

### 4. Study on the effect of adding Tween 20 and PEG 3350 on stabilization and precipitation prevention

| **Preparation** | **B4** | **B5** | **B6** | **B7** |
|---|---|---|---|---|
| Quetiapine fumarate | 5% | 5% | 5% | 5% |
| Anhydrous ethanol | 19.8% | 19.8% | 19.8% | 19.8% |
| Propylene glycol | 5% | 5% | 5% | 5% |
| PEG 3350 | 20% | 20% | 25% | 30% |
| Tween 20 | 1.5% | 3% | 1.5% | 1.5% |
| 0.1 M citric acid | Balance | | | |
| Preparation method: the individual components were weighed according to the formulation table, mixed, and dissolved sufficiently but were not completely dissolved. After standing, the supernatant was separated and collected. No precipitation occurred during 14 days of high and low temperature cycling, as well as after storage at room temperature for 4 months (low temperature: 2°C to 8°C; high temperature: 50°C). | | | | |

The experimental process and results demonstrated that Tween 20 and PEG 3350 both had a good stabilizing effect on the formulation solution, thereby preventing precipitation.

### 5. Study on the effect of adding surfactant and cyclodextrin on solubilization

| **Preparation** | **B8** | **B9** | **B10** |
|---|---|---|---|
| Quetiapine fumarate | 5% | 5% | 5% |
| HS-15 | 1.5% | / | / |
| HP-β-CD | / | 5% | 5% |
| Tween 20 | 2.5% | / | / |
| PEG 400 | 20% | 20% | 20% |
| Propylene glycol | 12% | 12% | 12% |
| Citrate buffer pair, pH 5.0 | Balance | | |
| **Saturation solubility (mg/mL)** | **15.04** | **28.87** | **26.61** |
| "/" indicates no addition of this component in the preparation. | | | |
| Preparation method: the individual components were weighed according to the formulation table, mixed, and dissolved sufficiently but were not completely dissolved. After standing, the supernatant was separated and collected. The saturation solubility of quetiapine in the supernatant was analyzed and determined (calculated as free base). | | | |

The experimental process and results demonstrated that the addition of cyclodextrin (HP-β-CD) had a certain improvement on the saturation solubility of quetiapine fumarate, but the improvement was still limited. Surfactants had only limited improvement on the saturation solubility of quetiapine fumarate. Further experiments also demonstrated that the addition of PEG 400 and surfactants was able to stabilize the formulation and prevent precipitation.

### 6. Study on the effect of adding DMA on solubilization

| **Preparation** | **B11** | **B12** | **B13** | **B14** |
|---|---|---|---|---|
| Quetiapine fumarate | 5.2% | 5% | 6.6% | 6.0% |
| DMA | 10% | 10% | 10% | 10% |
| PEG 400 | 15% | 17% | 17% | 17% |
| Propylene glycol | 12% | / | / | / |
| Anhydrous ethanol | 19.8% | 19% | 19% | 19% |
| 0.1 M citric acid | Balance | | | |
| **Saturation solubility (mg/mL)** | **45.18** | **43.68** | **57.40** | **52.81** |
| Recording of dissolution phenomenon | Slight precipitation at 2°C to 8°C for 1 day, and no precipitation at room temperature for 5 days | No precipitation at either 2°C to 8°C or room temperature for 5 days | Precipitation at 2°C to 8°C for 2 days, and a small amount of crystal precipitation at room temperature for 2 days | A small amount of precipitation at either 2°C to 8°C or room temperature for 1 day |
| "/" indicates no addition of this component in the preparation. | | | | |
| Preparation method: the individual components were weighed according to the formulation table, heated, and dissolved sufficiently. Quetiapine fumarate in each formulation was completely dissolved within 60 minutes on average. The saturation solubility of quetiapine in the solution was analyzed and determined (calculated as free base). | | | | |

The experimental process and results demonstrated that the addition of DMA significantly improved the saturation solubility of quetiapine fumarate, making it suitable for nasal spray administration. An active ingredient concentration greater than 50 mg/mL for nasal spray administration was able to significantly reduce the frequency of administration and thus reduce the corresponding nasal irritation.

### 7. Study on the effect of adding benzyl alcohol on solubilization

| **Preparation** | **B15** | **B16** |
|---|---|---|
| Quetiapine fumarate | 6.6% | 8.2% |
| Anhydrous ethanol | 19.8% | 19.8% |
| Benzyl alcohol | 5% | 10% |
| PEG 3350 | 20% | 20% |
| Tween 20 | 1.5% | 1.5% |
| 0.1 M citric acid | Balance | |
| **Saturation solubility (mg/mL)** | **57.65** | **70.87** |
| Preparation method: the individual components were weighed according to the formulation table, mixed, and dissolved sufficiently. The saturation solubility of quetiapine in the solution was analyzed and determined (calculated as free base). The clear supernatant from each formulation showed no precipitation after three months of storage at room temperature. | | |

| **Preparation** | **B17** | **B18** | **B19** | **B20** | **B21** | **B22** |
|---|---|---|---|---|---|---|
| Quetiapine fumarate | 7% | 7% | 5% | 7% | 5% | 6.7% |
| Propylene glycol | 12% | 12% | 12% | 12% | 5% | N/A |
| PEG 3350 | 5% | 5% | 5% | N/A | 20% | 20% |
| Benzyl alcohol | 5% | 5% | N/A | 5% | N/A | 5% |
| Tween 20 | 1.5% | 1.5% | 1.5% | 1.5% | 1.5% | 1.5% |
| Anhydrous ethanol | 10% | N/A | N/A | N/A | 19.8% | 19.8% |
| 0.1 M citric acid | Balance | 59.5% | 61.5% | Balance | Balance | Balance |
| PEG 400 | N/A | N/A | N/A | 15% | N/A | N/A |
| Purified water | N/A | Balance | Balance | N/A | N/A | N/A |

| Recording of dissolution phenomenon | Completely dissolved after stirring at room temperature for 10 minutes | Completely dissolved after heating and stirring at 50°C for 10 minutes | Not dissolved after heating and stirring at 50°C for 1 hour | Completely dissolved after heating and stirring at 50°C for approximately 20 minutes | Not dissolved after heating and stirring at 50°C for 1 hour | Completely dissolved after heating and stirring at 50°C for approximately 20 minutes |
|---|---|---|---|---|---|---|
| **Dissolved concentration (mg/mL)** | **Approximately 60** | **Approximately 60** | **< 43** | **Approximately 60** | **< 43** | **58** |
| "N/A" indicates no addition of this component in the preparation. | | | | | | |
| Preparation method: the individual components were weighed according to the formulation table, mixed, and dissolved sufficiently. The dissolution phenomenon was recorded, and the dissolved concentration was estimated (calculated as free base). | | | | | | |

The experimental process and results demonstrated that the addition of benzyl alcohol significantly improved the saturation solubility of quetiapine fumarate to more than 50 mg/mL, making it suitable for nasal spray administration. Furthermore, the saturation solubility is positively correlated with the amount of benzyl alcohol used. Compared with DMA, the addition of benzyl alcohol can achieve a higher saturation solubility while avoiding the drawback of severe nasal irritation caused by DMA.

### 8. Extended study on the effect of adding benzyl alcohol on solubilization

| **Preparation** | **B23** | **B24** | **B25** | **B26** | **B27** | **B28** |
|---|---|---|---|---|---|---|
| Quetiapine fumarate | 9.5% | 9.7% | 13.4% | 13.3% | 9.7% | 13% |
| Propylene glycol | 5% | 5% | 5% | 5% | 5% | 5% |
| PEG 3350 | 15% | 15% | 5% | 5% | 15% | 5% |
| Benzyl alcohol | 10% | 10% | 10% | 10% | 10% | 10% |
| Tween 20 | 1.5% | 1.5% | 1.5% | 1.5% | 1.5% | 1.5% |
| Pectin | / | 0.25% | / | 0.25% | 0.5% | 0.5% |
| Anhydrous ethanol | 19.8% | 19.8% | 19.8% | 19.8% | 19.8% | 19.8% |
| 0.1 M citric acid | Balance | | | | | |
| **Saturation solubility (mg/mL)** | **82.61** | **84.70** | **116.26** | **115.22** | **84.43** | **112.61** |
| "/" indicates no addition of this component in the preparation. | | | | | | |
| Preparation method: the individual components were weighed according to the formulation table, mixed, and dissolved sufficiently. The saturation solubility of quetiapine in the solution was analyzed and determined (calculated as free base). The clear supernatant from each formulation showed no crystal precipitation during 14 days of high and low temperature cycling, and after three months of storage at room temperature (low temperature: 2°C to 8°C; high temperature: 50°C). | | | | | | |

The experimental process and results demonstrated that on the basis of benzyl alcohol addition, a significantly enhanced saturation solubility of quetiapine was achieved. The additional addition of pectin at various levels was also able to maintain a relatively high saturation solubility. When PEG 3350 was reduced from 15% to 5% in the formulation, the saturation solubility showed a remarkable increasing trend, which may be attributed to the excessive levels of PEG 3350 occupying solubility capacity in the formulation. The possible reason for the increase in the solubility of the above formulation is that benzyl alcohol, propylene glycol and ethanol form a mixed system with stronger solubilization ability. Tween 20 and PEG 3350 mainly play a stabilizing role in the formulation, inhibiting quetiapine from precipitating under high and low temperature conditions.

### 9. Additional formulations with good solubilization effects prepared and validated in other embodiments

Based on the findings from the aforementioned solubilization studies, the amount of organic solvents used was reduced, and the following nasal spray preparations of solution-type quetiapine composition for intranasal administration were further prepared, all of which had a good high active ingredient concentration (the concentration in the table was estimated based on the complete dissolution of quetiapine fumarate, and the estimated concentration was calculated as free base).

| **Preparation** | **B29** | **B30** | **B31** | **B32** | **B33** | **B34** |
|---|---|---|---|---|---|---|
| Quetiapine fumarate | 7% | 7% | 6.5% | 6.5% | 6.5% | 7% |
| Propylene glycol | 6% | 6% | 6% | 6% | 6% | 6% |
| PEG 3350 | 5% | 5% | N/A | N/A | 5% | 5% |
| Benzyl alcohol | 5% | 5% | 5% | 5% | 5% | 5% |
| Tween 20 | 0.5% | N/A | 0.5% | N/A | N/A | N/A |
| 0.1 M citric acid | 50% | 50% | 50% | 50% | 50% | 1% |
| PEG 400 | N/A | N/A | 15% | 15% | 10% | N/A |
| Purified water | Balance | | | | | |

| **Dissolved concentration (mg/mL)** | **61** | **61** | **56** | **56** | **56** | **61** |
|---|---|---|---|---|---|---|
| "N/A" indicates no addition of this component in the preparation. | | | | | | |
| Preparation method: the individual components were weighed according to the formulation table, mixed, and dissolved sufficiently. The resulting clear solution was filled into a medium borosilicate glass vial, and assembled with a quantitative nasal spray pump to obtain the final product. | | | | | | |

### Example C: Study on the chemical stability of the solution-type intranasal administration composition of the present disclosure

### 1. Basic formulation design and stability study

| **Preparation** | **C1** | **C2** | **C3** | **C4** |
|---|---|---|---|---|
| Quetiapine fumarate | 7% | 5% | 5% | 7% |
| Propylene glycol | 6% | 6% | 6% | 12% |
| Benzyl alcohol | 5% | 5% | 5% | 7.5% |
| PEG 3350 | 5% | 2.5% | 2.5% | 2.5% |

| Water | Balance | | | |
|---|---|---|---|---|
| Citric acid monohydrate | 1% | 0.6% | 0.4% | N/A |
| pH | 3.9 | 4.1 | 4.2 | 4.9 |
| "N/A" indicates no addition of this component in the preparation. | | | | |
| Preparation method: the individual components were weighed according to the formulation table, mixed and dissolved until clear. The resulting solution was filled into a medium borosilicate glass vial, and assembled with a quantitative nasal spray pump to obtain the final product. | | | | |

The chemical stability of the above preparation (impurity G: 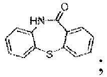 impurity H: 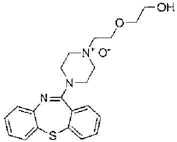 ) was investigated at 40°C and 75% RH. The analytical test data were as follows:

| **Preparation** | **Day 0** | **Day 12** | **Day 40** | |
|---|---|---|---|---|
| | **Total Impurities, %** | **Total Impurities, %** | **Impurity G, %** | **Impurity H, %** |
| Cl | 0.14 | 0.80 | 1.02 | 0.08 |
| C2 | 0.14 | 0.67 | 0.66 | 0.12 |
| C3 | 0.15 | 0.61 | 0.47 | 0.15 |
| C4 | 0.14 | 0.35 | 0.13 | 0.11 |

The results demonstrated that the above solution-type intranasal administration composition, which was prepared to increase saturation solubility, had good chemical stability.

### 2. Study on formulation stability with addition of a surfactant and an antioxidant

Based on the aforementioned C4 formulation, surfactants polyethylene glycol-15 hydroxystearate (HS-15) and Tween 80 were introduced. The pH of the solution was adjusted using NaOH to investigate their effect on the chemical stability of the formulation.

| **Preparation** | **C5** | **C6** | **C7** | **C8** | **C9** |
|---|---|---|---|---|---|
| Quetiapine fumarate | 7% | 7% | 7% | 7% | 7% |
| Propylene glycol | 12% | 12% | 12% | 12% | 12% |
| Benzyl alcohol | 7.5% | 7.5% | 7.5% | 7.5% | 7.5% |
| PEG 3350 | 2.5% | 2.5% | 2.5% | 2.5% | 2.5% |
| Water | Balance | | | | |
| HS-15 | / | 1.5% | / | / | / |
| Tween 80 | / | / | 1% | / | 0.5% |
| Sodium sulfite | / | / | / | 1% | 1% |
| Initial pH | 4.9 | 4.8 | 4.9 | 5.8 | 5.9 |
| pH adjusted with NaOH solution to: | 5.0 | 5.0 | 4.5 | 5.1 | 4.2 |
| "/" indicates no addition of this component in the preparation. | | | | | |
| Preparation method: the individual components were weighed according to the formulation table, mixed and dissolved until clear. The pH was adjusted using NaOH solution. The resulting solution was filled into a medium borosilicate glass vial, and assembled with a quantitative nasal spray pump to obtain the final product. | | | | | |

The above formulations were subjected to long-term (25°C, 75% RH) and accelerated (40°C, 75% RH) testing. The analytical test data were as follows:

| **Preparation** | **Total Impurity Content (%)** | | | **Content of Impurity G (%)** | |
|---|---|---|---|---|---|
| | **Day 0** | **25°C** | **40°C** | **25°C** | **40°C** |
| | | **Day 38** | **Day 38** | **Day 38** | **Day 38** |
| C5 | 0.07 | 0.09 | 0.17 | ND | 0.09 |
| C6 | 0.06 | 0.09 | 0.14 | ND | 0.07 |
| C7 | 0.07 | 0.11 | 0.39 | 0.04 | 0.18 |
| C8 | The total impurity content in C8 and C9 reached 0.84% and 2.09% respectively after 7 days of accelerated storage, and therefore no further testing was conducted. | | | | |
| C9 | | | | | |
| "ND" means not detected. | | | | | |

The results demonstrated that the stability of the formulation with the addition of surfactants HS-15 and Tween 80 was controllable and did not cause a significant increase in related substances. However, the introduction of the antioxidant sodium sulfite not only failed to improve the stability, but also unexpectedly led to a sharp decline in the stability of the formulation.

The use of NaOH to adjust the pH did not destroy the chemical stability. When the pH was appropriately increased, the formation of hydrolyzed impurity G was inhibited and the chemical stability was improved.

### 3. Study on formulation stability with introduction of an organic base

Increasing the pH of the formulation was found to inhibit the growth of impurity G. An attempt was made to introduce the organic base meglumine to increase the pH value of the formulation so as to enhance its stability.

| **Preparation** | **C10** | **C11** |
|---|---|---|
| Quetiapine fumarate | 7% | 7% |
| Propylene glycol | 12% | 12% |
| Benzyl alcohol | 7.5% | 7.5% |
| PEG 3350 | 2.5% | 2.5% |
| Water | Balance | |
| Meglumine | 0.25% | 0.50% |
| pH | 5.1 | 5.3 |
| Preparation method: the individual components were weighed according to the formulation table, mixed and dissolved until clear. The resulting solution was filled into a medium borosilicate glass vial, and assembled with a quantitative nasal spray pump to obtain the final product. | | |

The above formulations were subjected to 30-day long-term (25°C, 75% RH) and accelerated (40°C, 75% RH) testing. The analytical test data were as follows:

| **Preparation** | **Total Impurity Content (%)** | | | **Content of Impurity G (%)** | |
|---|---|---|---|---|---|
| | **Day 0** | **25**°C | **40**°C | **25**°C | **40**°C |
| | | **Day 30** | **Day 30** | **Day 30** | **Day 30** |
| C10 | 0.08 | 0.09 | 0.13 | ND | 0.05 |
| C11 | 0.08 | 0.09 | 0.12 | ND | 0.04 |
| "ND" means not detected. | | | | | |

The results demonstrated that the use of organic base meglumine to increase pH was beneficial to inhibiting the formation of hydrolyzed impurity G, thereby improving chemical stability.

### 4. Extended study on formulation stability

Firstly, stability studies were conducted with or without nitrogen filling, as well as the addition of or variation in the amount of the solubilizer HP-β-CD, the surfactant HS-15, and the stabilizer disodium edetate. The formulations were designed as follows:

| **Preparation** | **C12** | **C13** | **C14** | **C15** | **C16** | **C17** |
|---|---|---|---|---|---|---|
| Quetiapine fumarate | 7% | 7% | 7% | 7% | 7% | 7% |
| Propylene glycol | 12% | 12% | 12% | 12% | 12% | 12% |
| Benzyl alcohol | 7.5% | 7.5% | 7.5% | 7.5% | 7.5% | 7.5% |
| PEG 3350 | 2.5% | 2.5% | 2.5% | 2.5% | 2.5% | 2.5% |
| Water | Balance | | | | | |
| Meglumine | / | 0.5% | 0.5% | 0.5% | 0.5% | 0.5% |
| HS-15 | / | 3% | 3% | / | / | 3% |
| Disodium edetate | / | / | 0.05% | / | 0.05% | 0.05% |
| HP-β-CD | / | / | / | 5% | 5% | / |
| Sodium bisulfite | / | / | / | / | / | 0.5% |
| pH | 5.0 | 5.4 | 5.3 | 5.4 | 5.5 | 5.2 |
| "/" indicates no addition of this component in the preparation. | | | | | | |
| Preparation method: (1) the individual components were weighed according to the formulation table, mixed and dissolved until clear. The resulting clear solution was filled into a medium borosilicate glass vial, and assembled with a quantitative nasal spray pump to obtain the final product. (2) Parallel formulations, C16-1 and C17-1, were additionally prepared for C16 and C17, respectively, with an extra incorporation of nitrogen filled protection. | | | | | | |

The above formulations were subjected to 24-day long-term (25°C, 75% RH) and accelerated (40°C, 75% RH) testing. The analytical test data were as follows:

| **Preparation** | **Total Impurity Content (%)** | | | **Content of Impurity G (%)** | |
|---|---|---|---|---|---|
| | **Day 0** | **25**°C | **40**°C | **25**°C | **40**°C |
| | | **Day 24** | **Day 24** | **Day 24** | **Day 24** |
| C12 | 0.08 | 0.08 | 0.14 | ND | 0.06 |
| C13 | 0.08 | 0.09 | 0.12 | ND | 0.04 |
| C14 | 0.07 | 0.08 | 0.11 | ND | 0.04 |
| C15 | 0.07 | 0.10 | 0.11 | ND | 0.03 |
| C16 | 0.08 | 0.08 | 0.15 | ND | 0.03 |
| C16-1 | 0.08 | N/A | 0.14 | N/A | 0.03 |
| C17 | The total impurity content in C17 and C17-1 reached 0.62% and 0.38% respectively after 10 days of accelerated storage, and therefore no further testing was conducted. | | | | |
| C17-1 | | | | | |
| "N/A" means not applicable. | | | | | |
| "ND" means not detected. | | | | | |

The results demonstrate that the stability of the formulation with the addition of or variation in the amount of the solubilizer HP-β-CD, the surfactant HS-15, and the stabilizer disodium edetate was controllable and did not cause a significant increase in related substances. However, surprisingly, the introduction of the antioxidant sodium bisulfite not only failed to improve the antioxidant performance of quetiapine, but also unexpectedly led to a rapid decline in the stability of the formulation.

The results also demonstrated that there was no obvious difference in the impurity growth rate and content between the two formulations with and without nitrogen filling, that is, stable formulations could be obtained with or without nitrogen filling.

Based on above studies, the following formulations were further prepared, which are stable under long-term and accelerated test. The solution-type intranasal administration composition preparation used in further animal studies was also stable.

| **Preparation** | **C18** | **C19** | **C20** | **C21** | **C22** | **C23** | **C24** | **C25** | **C26** | **C27** | **C28** |
|---|---|---|---|---|---|---|---|---|---|---|---|
| Quetiapine fumarate | 0.25% | 1.7% | 6.7% | 1.7% | 6.7% | 1.7% | 6.7% | 1.7% | 6.7% | 1.7% | 6.7% |
| Propylene glycol | 12% | 11% | 11% | / | / | / | / | 11% | 11% | 11% | 11% |
| Ethanol | / | / | / | 19.8% | 19.8% | / | / | / | / | / | / |
| Glycerol | / | / | / | / | / | 2.2% | 2.2% | / | / | / | / |
| Benzyl alcohol | 1% | 3.2% | 7.5% | 3.2% | 7.5% | 3.2% | 7.5% | 3.2% | 7.5% | 3.2% | 7.5% |
| PEG 3350 | 2.5% | 5% | 5% | 5% | 5% | 5% | 5% | / | / | 5% | 5% |
| PEG 400 | / | / | / | / | / | / | / | 15% | 15% | / | / |
| Benzalkonium chloride | 0.02% | / | / | / | / | / | / | / | / | 0.02% | 0.02% |
| Meglumine | 0.02% | The pH value was adjusted with sodium hydroxide, meglumine, or citric acid. | | | | | | | | | |
| Water | Balance | | | | | | | | | | |
| pH | 6.1 | 5.3 | 5.3 | 5-6 | 5-6 | 5-6 | 5-6 | 5-6 | 5-6 | 5-6 | 5-6 |
| "/" indicates no addition of this component in the preparation. | | | | | | | | | | | |
| Preparation method: the individual components were weighed according to the formulation table, mixed and dissolved until clear. The resulting solution was filled into a medium borosilicate glass vial, and assembled with a quantitative nasal spray pump to obtain the final product. | | | | | | | | | | | |

The present disclosure is not limited to the above optional implementations, and various other forms of products may be derived by any person based on the teachings of the present disclosure. The above specific implementations should not be understood as limiting the scope of protection of the present disclosure. The scope of protection of the present disclosure should be based on the definition in the claims, and the description can be used to interpret the claims.

## Claims

1. A quetiapine composition for nasal administration, comprising quetiapine or a pharmaceutically acceptable salt thereof, wherein the composition is a solution.

2. The composition according to claim 1, wherein the mass percentage of quetiapine in the composition, calculated as free base, is from 0.22% to 11.7%, or from 4.3% to 11.7%,
or, preferably, quetiapine is dissolved in the composition in the form of fumarate, and the mass percentage of the quetiapine fumarate in the composition is from 0.25% to 13.4%, from 0.25% to 5%, from 0.25% to 1.7%, from 5% to 13.4%, from 5% to 9.7%, from 5% to 7%, 0.25%, 1.7%, 5%, 5.2%, 6%, 6.5%, 6.6%, 6.7%, 7%, 8.2%, 9.5%, 9.7%, 13%, 13.3% or 13.4%.

3. The composition according to claim 1, wherein the composition further comprises one or a combination of two or more selected from a solubilizer, a cosolvent, a viscosity enhancer, a stabilizer, a preservative, a pH regulator and a pH buffer.

4. The composition according to claim 3, wherein
the solubilizer optionally comprises one or a combination of two or more selected from benzyl alcohol, DMA, cyclodextrin and a surfactant, wherein the cyclodextrin optionally comprises one or a combination of two or more selected from β-cyclodextrin, hydroxypropyl-β-cyclodextrin and sulfobutyl-β-cyclodextrin, and wherein the surfactant optionally comprises one or a combination of two or more selected from Tween, Span, Carbomer and polyethylene glycol-15 hydroxystearate;
the cosolvent optionally comprises a saturated alcohol, and the saturated alcohol optionally comprises one or a combination of two or more selected from propylene glycol, ethanol and glycerol;
the viscosity enhancer optionally comprises one or a combination of two or more selected from polyethylene glycol, pectin, cellulose, polyolefin and polyacrylic acid;
the stabilizer optionally comprises edetate, and the edetate optionally comprises EDTA-2Na and/or EDTA-2K;
the preservative optionally comprises benzalkonium chloride, benzalkonium bromide, benzethonium chloride, potassium sorbate, methyl paraben, ethyl paraben, propyl paraben, butyl paraben, isopropyl paraben, isobutyl paraben, sodium propyl paraben and/or sodium methyl paraben;
the pH regulator optionally comprises one or a combination of two or more selected from citric acid, hydrochloric acid, sodium hydroxide, potassium hydroxide and meglumine; and
the pH buffer optionally comprises one or a combination of two or more selected from citrate buffer, phosphate buffer and acetate buffer.

5. The composition according to claim 1, wherein the composition further comprises a solubilizer, a cosolvent, a viscosity enhancer and water,
the solubilizer is benzyl alcohol, and the mass percentage of the benzyl alcohol in the composition is selected from 1% to 10%, from 1% to 9.3%, from 5% to 9.3%, 1%, 3.2%, 5%, 7.5% or 9.3%;
the cosolvent is one or a combination of two or more selected from propylene glycol, ethanol and glycerol, preferably propylene glycol, the mass percentage of the propylene glycol in the composition is selected from 0% to 12%, from 4.6% to 12%, from 6% to 12%, 4.6%, 4.7%, 5%, 6%, 10%, 11% or 12%, the mass percentage of ethanol in the composition is selected from 0% to 20%, from 6% to 20%, from 0% to 19.8%, from 18.3% to 19.8%, 6%, 10%, 18.3%, 18.5% or 19.8%, and the mass percentage of glycerol in the composition is selected from 0% to 5%, from 0% to 3%, from 0% to 2.5%, from 0% to 2.2% or 2.2%; and
the viscosity enhancer is polyethylene glycol, preferably PEG 3350 or PEG 400, the mass percentage of PEG 3350 in the composition is selected from 0% to 20%, from 2.5% to 15%, from 5% to 15%, 2.5%, 4.6%, 4.7%, 5%, 15% or 20%, and the mass percentage of PEG 400 in the composition is selected from 0% to 15%, 10% or 15%.

6. The composition according to claim 5, wherein the composition further comprises one or a combination of two or more selected from a stabilizer, a preservative, a pH regulator and a pH buffer,
wherein the stabilizer is EDTA-2Na, and the mass percentage of EDTA-2Na in the composition is selected from 0% to 0.1% or 0.05%;
the preservative is benzalkonium chloride, and the mass percentage of the benzalkonium chloride in the composition is selected from 0% to 0.02% or 0.02%;
the pH regulator is one or a combination of two or more selected from citric acid, hydrochloric acid, sodium hydroxide and meglumine, and the pH regulator adjusts the pH value of the composition to from 4.1 to 6.0, from 4.5 to 6.0, from 5.0 to 6.0, and 4.1, 4.5, 4.9, 5.0, 5.1, 5.3 or 6.0; and
the pH buffer is a citrate buffer, a phosphate buffer or an acetate buffer.

7. An intranasal administration composition with improved dissolved concentration of quetiapine, wherein the composition comprises quetiapine or a pharmaceutically acceptable salt thereof, wherein the composition is a solution and further comprises a solubilizer.

8. The composition according to claim 7, wherein the solubilizer comprises one or a combination of two or more selected from benzyl alcohol, DMA, cyclodextrin and a surfactant.

9. The composition according to claim 7, wherein the solubilizer comprises benzyl alcohol and/or DMA.

10. The composition according to claim 7, wherein the solubilizer comprises benzyl alcohol, the mass percentage of benzyl alcohol in the composition is selected from 1% to 10%, from 3.2% to 10%, from 5% to 10%, from 5% to 9.3%, from 5% to 7.5%, 1%, 3.2%, 5%, 7.5%, 9.3% or 10%, and optionally, the mass ratio of benzyl alcohol to quetiapine in the composition is from 1: 0.8 to 1: 1.2.

11. The composition according to claim 10, wherein
the solubilizer in the composition may only comprises benzyl alcohol,
or the solubilizer in the composition may only comprises benzyl alcohol and cyclodextrin,
or the solubilizer in the composition may only comprises benzyl alcohol and the surfactant,
or the solubilizer in the composition may only comprises benzyl alcohol, cyclodextrin and the surfactant.

12. The composition according to claim 10, wherein the composition further comprises a cosolvent, wherein the cosolvent optionally comprises saturated alcohol, the saturated alcohol optionally comprises one or a combination of two or more selected from propylene glycol, ethanol and glycerol, and the saturated alcohol is preferably propylene glycol or a combination of propylene glycol and ethanol;
and wherein the mass percentage of propylene glycol in the composition is selected from 0% to 12%, from 4.6% to 12%, from 6% to 12%, 4.6%, 4.7%, 5%, 6%, 10%, 11% or 12%;
the mass percentage of ethanol in the composition is selected from 0% to 20%, from 6% to 20%, from 0% to 19.8%, from 18.3% to 19.8%, 6%, 10%, 18.3%, 18.5% or 19.8%; and
the mass percentage of glycerol in the composition is selected from 0% to 5%, from 0% to 3%, from 0% to 2.5%, from 0% to 2.2% or 2.2%.

13. The composition according to claim 10, wherein the composition further comprises a surfactant and/or polyethylene glycol, preferably further comprises one or a combination of two or more selected from Tween, PEG 3350 and PEG 400, the Tween comprises Tween 80 or Tween 20;
wherein the mass percentage of PEG 3350 in the composition is selected from 0% to 20%, from 2.5% to 15%, from 5% to 15%, 2.5%, 4.6%, 4.7%, 5%, 15% or 20%;
the mass percentage of PEG 400 in the composition is selected from 0% to 15%, 10% or 15%; and
the mass percentage of Tween in the composition is selected from 0% to 1.5%, 0.5%, 1%, 1.4% or 1.5%.

14. The composition according to claim 8, wherein the cyclodextrin comprises one or a combination of two or more selected from β-cyclodextrin, hydroxypropyl-β-cyclodextrin and sulfobutyl-β-cyclodextrin, and the mass percentage of cyclodextrin in the composition is selected from 0% to 10%, from 1% to 5%, or 5%.

15. The composition according to claim 7, wherein the dissolved concentration of quetiapine in the composition, calculated as free base, is from 4.3% to 11.7% or from 5.6% to 11.7% by mass;
or, preferably, quetiapine is dissolved in the composition in the form of fumarate, and the mass percentage of quetiapine fumarate in the composition is from 5% to 13.4%, from 5% to 9.7%, from 5% to 7%, 5%, 5.2%, 6%, 6.5%, 6.6%, 6.7%, 7%, 8.2%, 9.5%, 9.7%, 13%, 13.3% or 13.4%.

16. The composition according to any one of claims 7 to 15, wherein the composition further comprises water, and one or a combination of two or more selected from a viscosity enhancer, a stabilizer, a preservative, a pH regulator and a pH buffer.

17. A stable intranasal administration composition, wherein the composition is a solution comprising water and the following components:
| **Component** | **Mass Percentage** |
|---|---|
| Quetiapine or a pharmaceutically acceptable salt thereof (calculated as free base) | 0.22%-11.7% |
| Solubilizer | 1%-10% |
| Cosolvent | 0%-20% |
| Viscosity enhancer | 0%-15% |

18. The composition according to claim 17, wherein the mass percentage of quetiapine in the composition, calculated as free base, is from 4.3% to 11.7%;
or, preferably, quetiapine is dissolved in the composition in the form of fumarate, and the mass percentage of quetiapine fumarate in the composition is from 0.25% to 13.4%, from 0.25% to 5%, from 0.25% to 1.7%, from 5% to 13.4%, from 5% to 9.7%, from 5% to 7%, 0.25%, 1.7%, 5%, 5.2%, 6%, 6.5%, 6.6%, 6.7%, 7%, 8.2%, 9.5%, 9.7%, 13%, 13.3% or 13.4%.

19. The composition according to claim 17, wherein
the solubilizer comprises one or a combination of two or more selected from benzyl alcohol, cyclodextrin, Tween and polyethylene glycol-15 hydroxystearate, the cyclodextrin optionally comprises hydroxypropyl-β-cyclodextrin, and the Tween optionally comprises Tween 80 and/or Tween 20;
and wherein the mass percentage of benzyl alcohol in the composition is selected from 1% to 10%, from 1% to 9.3%, from 5% to 9.3%, 1%, 3.2%, 5%, 7.5% or 9.3%, the mass percentage of cyclodextrin in the composition is selected from 0% to 5% or 5%, the mass percentage of Tween in the composition is selected from 0% to 1.4%, 1% or 1.4%, and the mass percentage of polyethylene glycol-15 hydroxystearate in the composition is selected from 0% to 3%, 1.5% or 3%.

20. The composition according to claim 17, wherein
the cosolvent comprises one or a combination of two or more selected from propylene glycol, ethanol and glycerol;
and wherein the mass percentage of propylene glycol in the composition is selected from 0% to 12%, from 4.6% to 12%, from 6% to 12%, 4.6%, 4.7%, 6%, 11% or 12%, the mass percentage of ethanol in the composition is selected from 0% to 20%, from 0% to 19.8%, from 18.3% to 19.8%, 18.3%, 18.5% or 19.8%, and the mass percentage of glycerol in the composition is selected from 0% to 5%, from 0% to 3%, from 0% to 2.5%, from 0% to 2.2% or 2.2%.

21. The composition according to claim 17, wherein
the viscosity enhancer comprises polyethylene glycol and/or pectin, and the polyethylene glycol optionally comprises PEG 3350 and/or PEG 400;
and wherein the mass percentage of PEG 3350 in the composition is selected from 0% to 5%, 2.5% to 5%, 2.5%, 4.6%, 4.7% or 5%, the mass percentage of PEG 400 in the composition is selected from 0% to 15% or 15%, and the mass percentage of pectin in the composition is selected from 0% to 1% or 0.7%.

22. The composition according to claim 17, wherein the composition further comprises any one or a combination of two or more selected from the following components:
| Component | Mass Percentage |
|---|---|
| Stabilizer | 0%-0.1% |
| Preservative | 0%-0.2% |
| pH regulator | An amount effective to adjust the pH of the composition to 4.1-6.9 |

23. The composition according to claim 22, wherein
the stabilizer comprises edetate, the edetate comprises disodium edetate and/or dipotassium edetate, and the mass percentage of the disodium edetate in the composition is selected from 0% to 0.1% or 0.05%;
the preservative comprises benzalkonium chloride, and the mass percentage of benzalkonium chloride in the composition is selected from 0% to 0.02% or 0.02%;
the pH regulator comprises one or a combination of two or more selected from sodium hydroxide, potassium hydroxide, meglumine, citric acid and hydrochloric acid, and the pH regulator is added in an amount effective to adjust the pH of the composition to a value selected from 4.1 to 6.0, from 4.5 to 6.0, from 5.0 to 6.0, 4.1, 4.5, 4.9, 5.0, 5.1, 5.3 or 6.0, and wherein the mass percentage of meglumine in the composition is selected from 0% to 0.5%, 0.02%, 0.25% or 0.5%, and the citric acid is selected as a 1 M citric acid solution.

24. The composition according to claim 23, wherein optionally, a pH buffer is further added to or not added to the composition, and the pH buffer optionally comprises one or a combination of two or more selected from citrate buffer, phosphate buffer and acetate buffer.

25. The composition according to claim 17, wherein
the composition is free of sodium sulfite and sodium bisulfite.

26. The composition according to claim 17, wherein
the composition consists of quetiapine fumarate, a solubilizer, a cosolvent, a viscosity enhancer and water,
the solubilize is benzyl alcohol,
the cosolvent is one or a combination of two or more selected from propylene glycol, ethanol and glycerol, preferably propylene glycol,
the viscosity enhancer is PEG 3350 and/or PEG 400; and
optionally, one or a combination of two or more selected from benzalkonium chloride, a pH regulator and a pH buffer is further added to or not added to the composition.

27. A quetiapine nasal spray, comprising the composition according to any one of claims 1 to 26 and a quantitative nasal spray device for applying and delivering the composition into the nasal cavity, wherein a volume of the composition delivered per spray by the quantitative nasal spray device is selected from 25 µL to 200 µL, from 50 µL to 150 µL, 25 µL, 50 µL, 75 µL, 100 µL, 125 µL, 150 µL, 175 µL or 200 µL.

28. Use of the composition according to any one of claims 1 to 26 or the quetiapine nasal spray according to claim 27 in the manufacture of a medicament for treating mental diseases.
